# EUROPEAN PATENT APPLICATION

(11) **EP 4 498 087 A1**
(43) Date of publication of application: **29.01.2025**
(21) Application number: 23774764.7
(22) Date of filing: 16.03.2023
(51) Int. Cl.: G01N 33/543

(54) **IMMUNOCHROMATOGRAPHIC INSPECTION APPARATUS**

(30) Priority: 22.03.2022 JP 2022046017
(71) Applicant: FUJIFILM Corporation, Tokyo 106-8620 (JP)
(72) Inventor: USHIKURA, Shinichi, Ashigarakami-gun, Kanagawa 258-8538 (JP)
(74) Representative: Dehns Germany Partnerschaft mbB
(86) International application number: PCT/JP2023/010399
(87) International publication number: WO 2023/182168

(57) **Abstract**

An immunochromatographic assay apparatus includes a loading part into which a cartridge is attachably and detachably loaded, the cartridge including a carrier having a spotting region where a sample is spotted and an assay region where a color development state changes depending on whether the sample is positive or negative; and a processor that performs a main determination, which is a determination of whether the sample is positive or negative, based on an assay region image of the assay region captured by an image sensor, in which the processor divides the assay region image into a plurality of areas extending in the row direction, derives an average value or a center value in each column in the areas using at least a part of the plurality of pixels included in each of the columns, and generates an area profile in the row direction for each of the areas using the derived average value or center value, and executes, in the main determination, condition determination processing using the area profile generated for each of the areas.

## Description

### BACKGROUND OF THE INVENTION

### 1. Field of the Invention

The present invention relates to an immunochromatographic assay apparatus.

### 2. Description of the Related Art

Among immunoassay methods, an immunochromatographic method is generally widely utilized, because operation is easy and assay can be performed within a short period of time.

In the immunochromatographic method, an immunochromatographic carrier provided with an assay region on which an antibody that specifically binds to an antigen, which is a test substance, is immobilized is used. In a case where a labeled antibody that specifically binds to the antigen is developed on an immunochromatographic carrier together with a sample containing the antigen, the antigen binds to the antibody immobilized on the assay region and the labeling substance is captured via the antigen. Since the assay region develops a color from the labeling substance captured in the assay region, the sample is determined as positive in a case where a color optical density of the assay region is equal to or more than a reference value.

JP2009-115470A discloses an assay apparatus that optically analyzes a color development state of an assay region of an immunochromatographic carrier. The assay apparatus includes a sensor that optically detects a reaction state and a measurement unit that performs a determination of whether the sample is positive or negative based on a detection result. In JP2009-115470A, in order to suppress an occurrence of an erroneous determination due to deterioration of the immunochromatographic carrier, presence or absence of the deterioration of the immunochromatographic carrier is determined before the determination. At this time, in a state where the sample is developed, a change amount in brightness in a predetermined region on the immunochromatographic carrier is measured, and in a case where the change amount in brightness is larger than a predetermined standard value, it is determined that the immunochromatographic carrier has deteriorated. Accordingly, the erroneous determination can be suppressed, which is due to the deterioration of the immunochromatographic carrier.

### SUMMARY OF THE INVENTION

In an assay apparatus, a sample may be determined as positive regardless of being originally negative by color development due to non-specific adsorption of a labeling substance in an assay region or in the vicinity of the assay region, in addition to an erroneous determination due to deterioration of an immunochromatographic carrier. Such an erroneous determination due to the non-specific adsorption of the labeling substance impairs a reliability of an immunochromatographic assay. In the assay apparatus, it is desired that an assay result with the higher reliability than that in the related art is presented.

The present disclosure has been made in view of the above-described circumstances, and an object of the present disclosure is to provide an immunochromatographic assay apparatus that can present an assay result with the higher reliability than that in the related art.

According to an aspect of the present disclosure, there is provided an immunochromatographic assay apparatus comprising:
a loading part into which a cartridge is attachably and detachably loaded, the cartridge including a carrier having a spotting region where a sample is spotted and an assay region where a color development state changes depending on whether the sample is positive or negative;
an image sensor that captures an observation region including the assay region and outputs an observation image including the observation region; and
a processor that performs a main determination, which is a determination of whether the sample is positive or negative, based on an assay region image of the assay region captured by the image sensor,
in which in a case where a development direction of the sample in the carrier is set as a row direction and a direction intersecting the row direction is set as a column direction, the assay region is a line-like region extending along the column direction,
the assay region image is an image in which a plurality of pixels are two-dimensionally arranged in a matrix shape,
the processor divides the assay region image into a plurality of areas extending in the row direction, derives an average value or a center value in each column in the areas using at least a part of the plurality of pixels included in each of the columns, and generates an area profile in the row direction for each of the areas using the derived average value or center value, and
executes, in the main determination, condition determination processing using the area profile derived for each of the areas.

In the immunochromatographic assay apparatus according to an aspect of the present disclosure,
the processor may execute, as the condition determination processing, at least one of first condition determination processing, second condition determination processing, or third condition determination processing for determining whether or not a value derived based on the area profile satisfies a preset condition, determine that the sample is negative in a case where even one of the conditions is not satisfied in the executed condition determination processing, or determine that the sample is positive in a case where all the conditions are satisfied in the executed condition determination processing,
the first condition determination processing may be condition determination processing, in which a differential area profile obtained by differentiating the area profile for each of the areas is used, a position in the row direction indicating a highest maximal value in the differential area profile is derived for each of the areas, a standard deviation at a plurality of derived positions in the row direction is derived, and whether or not a first condition that the standard deviation is less than a preset first threshold value is satisfied is determined,
the second condition determination processing may be condition determination processing, in which the differential area profile for each of the areas is added, a difference between a highest maximal value in an added addition differential area profile and an average value of maximal values other than the highest maximal value is derived, and whether or not a second condition that the difference is larger than a preset second threshold value is satisfied is determined, and
the third condition determination processing may be condition determination processing, in which the area profile for each of the areas is added, and whether or not a third condition that a value at a position in a row direction preset in an addition area profile is larger than a third threshold value is satisfied is determined.

In the immunochromatographic assay apparatus according to an aspect of the present disclosure, the processor may execute, as the condition determination processing, all of the first condition determination processing, the second condition determination processing, and the third condition determination processing.

In the immunochromatographic assay apparatus according to an aspect of the present disclosure,
the processor may determine, in the main determination, a representative value of each of the columns of the observation image before the condition determination processing, generates an observation region profile in the row direction based on the representative value of each of the columns, and extracts an assay region profile corresponding to the assay region from the generated observation region profile,
determine that the sample is negative in a case where a maximum position, which is a position in the row direction indicating a maximum density in the assay region profile, is located in any of both end regions of the assay region profile,
derive a center position of the assay region in the row direction and a width of the assay region, which are determined based on the maximum density and the maximum position, in a case where the maximum position is not located in any of both the end regions,
execute pre-condition determination processing, in which whether or not a pre-determination condition that the maximum density is equal to or more than a preset fourth threshold value and each of the center position and the width is within a preset range is satisfied is determined, and
determine that the sample is negative in a case where the pre-determination condition is not satisfied, and execute the condition determination processing in a case where the pre-determination condition is satisfied.

In the immunochromatographic assay apparatus according to an aspect of the present disclosure,
the processor may determine, in the main determination, whether or not the maximum density is equal to or more than a preset fifth threshold value larger than the fourth threshold value before the condition determination processing is executed in a case where it is determined that the pre-determination condition is satisfied in the pre-condition determination processing, determine that the sample is positive in a case where the maximum density is equal to or more than the fifth threshold value, and execute the condition determination processing in a case where the maximum density is less than the fifth threshold value.

With the immunochromatographic assay apparatus according to an aspect of the present disclosure, an assay result with higher reliability than that in the related art can be presented.

### BRIEF DESCRIPTION OF THE DRAWINGS

Fig. 1 is a perspective view showing an appearance of an immunochromatographic assay apparatus according to an embodiment.
Fig. 2 is a perspective view of a cartridge.
Fig. 3 is an exploded perspective view of the cartridge.
Fig. 4 is a diagram showing a positional relationship among an assay strip, a multifunctional member, a first reagent holding part, and a second reagent holding part, in the cartridge.
Fig. 5 is an explanatory diagram of an immunochromatographic method.
Fig. 6 is a partially broken side view of the assay apparatus in a state where the cartridge is loaded.
Fig. 7 is a diagram (part 1) showing a processing procedure of a main determination.
Fig. 8 is a diagram (part 2) showing the processing procedure of the main determination.
Fig. 9 is an explanatory diagram of a method of determining a representative value.
Fig. 10 is an observation region profile based on the representative value.
Fig. 11 is a diagram showing steps of extracting an assay region profile, exploring for a position showing a maximum density difference, and defining a line position and a line width.
Fig. 12 is an explanatory diagram of an example in which an observation image is divided into a plurality of areas.
Fig. 13 is a diagram showing an area profile generated by dividing an assay region image into four parts.
Fig. 14 is a diagram showing a differential profile of the area profile shown in Fig. 13.
Fig. 15 is a diagram showing an addition differential profile to which the differential profile of Fig. 14 is added.
Fig. 16 is a diagram showing an addition area profile to which the area profile shown in Fig. 13 is added.

### DESCRIPTION OF THE PREFERRED EMBODIMENTS

Embodiments of the immunochromatographic assay apparatus according to an aspect of the present disclosure will be described with reference to the drawings.

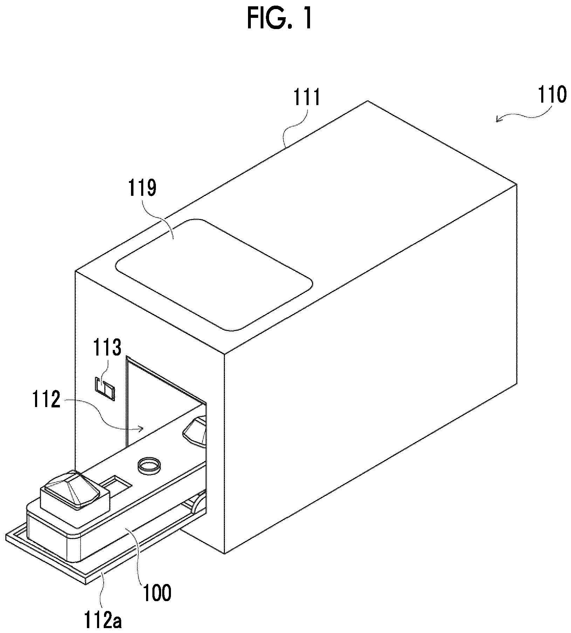

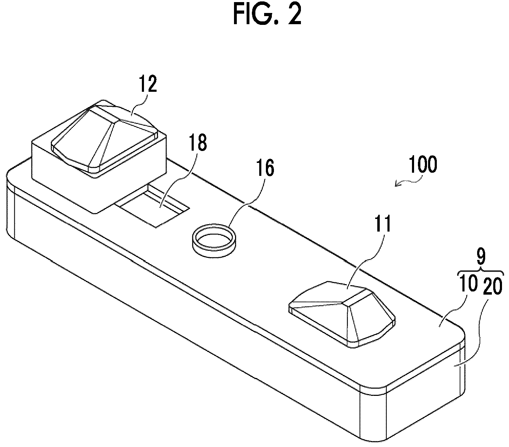

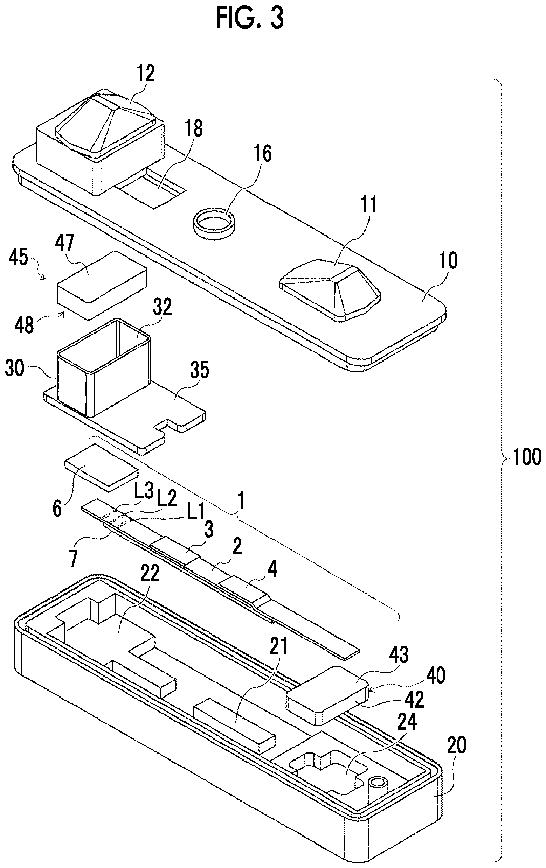

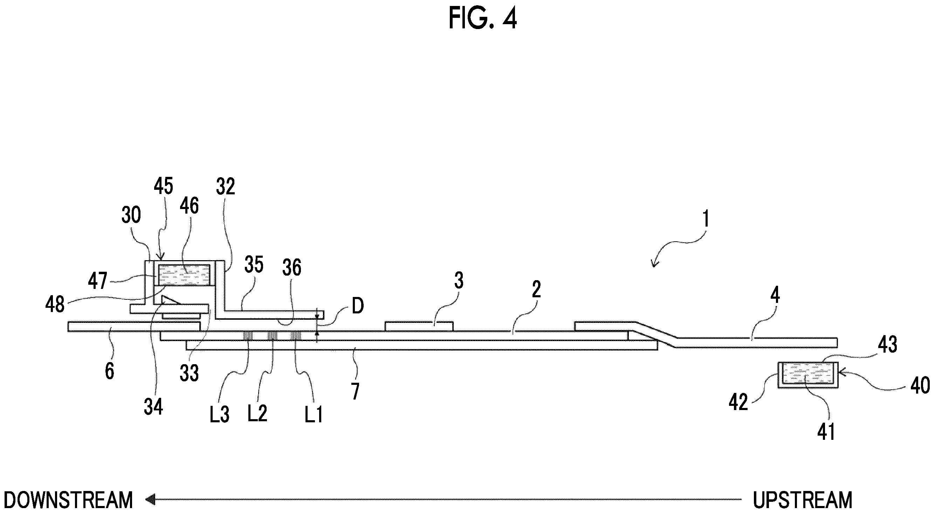

Fig. 1 is a perspective view showing an appearance of an immunochromatographic assay apparatus 110 (hereinafter, simply referred to as an assay apparatus 110) according to an embodiment. Fig. 2 is an external view of a cartridge 100 mounted on the assay apparatus 110 and Fig. 3 is an exploded perspective view of the cartridge 100. Fig. 4 is a diagram showing a positional relationship of main accommodated components in the cartridge 100.

The cartridge 100 is a single-use type that is used one by one in each sample, which is an assay target. As shown in Fig. 3, an assay strip 1 including an immunochromatographic carrier 2 (hereinafter, referred to as a carrier 2) is provided in the cartridge 100. An assay region L1 is provided in the carrier 2, and a color development state changes depending on whether or not the sample contains a test substance, that is, whether the sample is positive or negative.

The sample is simply required to be a specimen with a possibility in which a test substance is contained, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, a liquid specimen containing animals and plants themselves or a dried body thereof. Examples of the test substance include an antigen, an antibody, a protein, and a low-molecular-weight compound.

In the assay apparatus 110 of the present example, the cartridge 100 in a state in which the sample is spotted is loaded. Then, the assay apparatus 110 detects a color development state of the assay region L1 of the loaded cartridge 100, determines whether the sample is positive or negative, and presents the assay result. Hereinafter, a determination of whether the sample is positive or negative will be referred to as a main determination. In a case where a plurality of samples are assayed, the cartridge 100 for each sample is loaded one by one into the assay apparatus 110. Such cartridge 100 is also referred to as an immunochromatographic assay tool, an immunochromatographic assay kit, or the like.

As shown in Fig. 1, the assay apparatus 110 comprises a case body 111, and the case body 111 comprises a loading part 112 in which the cartridge 100 is attachably and detachably loaded. As an example, an opening for inserting the cartridge 100 into the case body 111 and an opening/closing lid 112a for opening and closing the opening are provided on the front surface of the case body 111. The opening/closing lid 112a is opened when the cartridge 100 is loaded, the cartridge 100 is inserted into the case body 111, and the opening/closing lid 112a is closed after the cartridge 100 has been loaded into the loading part 112. The assay is performed in a state where the opening/closing lid 112a is closed.

In addition, a power switch 113 is provided on the front surface of the case body 111, and a monitor 119 is provided on an upper surface of the case body 111. An assay result, an error message or the like is displayed on the monitor 119. As an example, the monitor 119 is a touch panel monitor, and various operation screens are displayed. Through the operation screen, the user can input a start instruction of processing and an operation instruction such as selection of an assay procedure.

As shown in Fig. 2 and Fig. 3, as an example, the cartridge 100 comprises a housing 9 configured with a case member 20 and a cover member 10. The housing 9 is formed of, for example, a resin material. An opening is formed in an upper part of the case member 20, and in addition to the assay strip 1, a first reagent holding part 40, a second reagent holding part 45, and the like are accommodated therein. The cover member 10 covers the opening of the case member 20 by being attached to an opening portion of the case member 20. The housing 9 has an elongated shape as a whole in accordance with an elongated shape of the assay strip 1.

In the present example, a dropping port 16, an observation window 18, a first pressed part 11, and a second pressed part 12 are provided on an upper part of the housing 9 configured with the cover member 10. Each of these parts is integrally molded with the cover member 10 as an example. The dropping port 16 is an opening for adding dropwise a sample into an inside of the housing 9. A boss is vertically provided on an edge of the dropping port 16 toward an upper part. The observation window 18 is a window for observing the assay region L1 from the outside, and is formed of a transparent member as an example. In the present example, the size of the observation window 18 is a size such that, in addition to the assay region L1, a control region L2 and a color development region L3, which will be described later, can also be observed.

The first pressed part 11 is an operating part operated to supply a first reagent 41 (see Fig. 4) in the first reagent holding part 40 to the carrier 2. The second pressed part 12 is an operating part operated to supply a second reagent 46 in the second reagent holding part 45 to the carrier 2. As will be described later, the first reagent 41 and the second reagent 46 are amplification agents for amplifying the color development in the assay region L1 in a case where a sample 50 is positive.

In a case where a pressing force is applied from the outside as an external force to the first pressed part 11, the first pressed part 11 is deformed. As an example, the first pressed part 11 has a quadrangular pyramid shape, and in a case where a pressing force is applied from above to a region including an apex of the quadrangular pyramid, the first pressed part 11 is deformed such that the apex of the quadrangular pyramid sinks into the inside of the housing 9. In a case where the first pressed part 11 is deformed in this manner, a pressing force is applied to the first reagent holding part 40 inside the housing 9. In the first reagent holding part 40, deformation or the like due to a pressing force applied through the first pressed part 11 occurs. Due to this deformation or the like, the first reagent 41 held by the first reagent holding part 40 is supplied to the assay strip 1.

In addition, it is preferable that the first pressed part 11 is deformed by pressing and then the deformed state is maintained. The reason is as follows. As will be described later, in the assay apparatus 110 of the present example, the cartridge 100 in a state in which the first pressed part 11 is pressed in advance by the user can be loaded. This is because, in a case where the first pressed part 11 is pressed by the user before being loaded into the assay apparatus 110, the first pressed part 11 in which the deformation is maintained even after the user releases the hand, is easier to continue the supply of the first reagent 41.

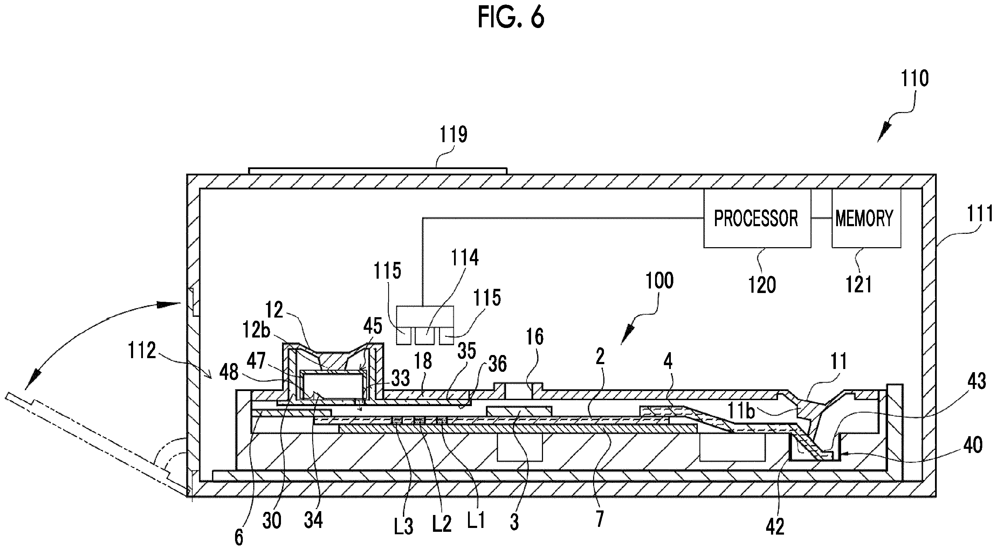

Similarly, in a case where a pressing force is applied from the outside as an external force to the second pressed part 12, the second pressed part 12 is deformed. Similarly to the first pressed part 11, the second pressed part 12 of the present example also has a quadrangular pyramid shape, and in a case where a pressing force is applied from above to a region including an apex of the quadrangular pyramid, the second pressed part 12 is deformed such that the apex of the quadrangular pyramid sinks into the inside of the housing 9. In a case where the second pressed part 12 is deformed in this manner, a pressing force is applied to the second reagent holding part 45 inside the housing 9. In the second reagent holding part 45, deformation or the like due to a pressing force applied through the second pressed part 12 occurs. Due to this deformation or the like, the second reagent 46 held by the second reagent holding part 45 is supplied to the assay strip 1. In the second pressed part 12 of the present example, an abutting part 12b that abuts the second reagent holding part 45 is provided (see Fig. 6).

As will be described later, in the assay apparatus 110 of the present example, a plurality of assay flows can be selected. In a case where in the assay apparatus 110, the assay flow for supplying the second reagent 46 is selected from the assay flows that can be selected in the assay apparatus 110, the second pressed part 12 is pressed by an internal mechanism of the assay apparatus 110. Therefore, the second pressed part 12 may be pressable by the internal mechanism. In a case of selecting an assay flow in which the cartridge 100 is loaded into the assay apparatus 110 after the supply of the first reagent 41 and the second reagent 46, the cartridge 100 is preferably an aspect that the second pressed part 12 is also pressable by the user.

As shown in Fig. 3 and Fig. 4, the case member 20 accommodates the assay strip 1 including the carrier 2 along a longitudinal direction. In the case member 20, the first reagent holding part 40 is disposed on one end part side (an upstream side shown in Fig. 4) in the longitudinal direction. In the case member 20, in a portion where the first reagent holding part 40 is disposed, a first accommodating part 24 having a recessed shape in accordance with a shape of the first reagent holding part 40 is formed. One end part of the assay strip 1 is disposed above the first reagent holding part 40 in a state of being accommodated in the first accommodating part 24.

The first reagent holding part 40 holds the first reagent 41. The first reagent holding part 40 is configured with, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward. The pressing force applied from the first pressed part 11 is transmitted to the sheet member 43 of the first reagent holding part 40 via the end part of the assay strip 1 to break the sheet member 43 (see Fig. 6). The sheet member 43 is broken, and thus the first reagent 41 is supplied to the assay strip 1. In the first pressed part 11 of the present example, a protruding part 11b that abuts the sheet member 43 is provided (see Fig. 6). The protruding part 11b has, for example, an elongated shape extending in a longitudinal direction in a width direction of the assay strip 1 and a pointed shape tapering toward the sheet member 43, such that the sheet member 43 is easily broken.

In addition, the cartridge 100 comprises a multifunctional member 30 having a function of accommodating the second reagent holding part 45. The multifunctional member 30 is disposed on the other end part side (a downstream side shown in Fig. 4) of the case member 20 and above the assay strip 1. The multifunctional member 30 is a member in which a second accommodating part 32 and a flow channel forming part 35 are integrally formed. The second accommodating part 32 is a part accommodating the second reagent holding part 45. The second accommodating part 32 has a box shape having an opened upper surface. As shown in Fig. 4, on a bottom portion of the second accommodating part 32, a protrusion part 34 for breaking a sheet member 48, which will be described later, of the second reagent holding part 45, and an opening 33 that allows flowing the second reagent 46 flowed out from the second reagent holding part 45, toward the carrier 2 are formed.

The flow channel forming part 35 is provided to be connected to the upstream side from the second accommodating part 32. The flow channel forming part 35 has a flat plate shape, is disposed at a position facing the assay region L1 or the like in a longitudinal direction of the carrier 2, and is disposed with an interval from the carrier 2. Then, between the flow channel forming part 35 and the carrier 2, a flow channel for flowing the second reagent 46 flowed out from the second accommodating part 32 toward the assay region L1 or the like is formed. In this way, the flow channel forming part 35 is disposed between the observation window 18 and the assay region L1 or the like of the carrier 2. Therefore, the flow channel forming part 35 is formed of a transparent member and thus the assay region L1 or the like can be observed through the observation window 18.

The second reagent holding part 45 holds the second reagent 46. The second reagent holding part 45 is configured with, for example, a container 47 formed of a resin material and having an opening on one surface, and the sheet member 48 that covers the opening of the container 47 and is breakable. The container 47 is filled with the second reagent 46, and the opening of the container 47 is sealed by the sheet member 48. The second reagent holding part 45 is disposed in the second accommodating part 32 in a posture in which the sheet member 48 faces downward. Accordingly, the sheet member 48 faces the protrusion part 34 in the second accommodating part 32.

The pressing force applied from the second pressed part 12 to the second reagent holding part 45 acts in a direction of pushing down the second reagent holding part 45 downward. Accordingly, the sheet member 48 is pressed against the protrusion part 34. The sheet member 48 is pressed against the protrusion part 34 to break the sheet member 48 (see Fig. 6). The sheet member 48 is broken, and thus the second reagent 46 is supplied to the carrier 2 through the flow channel formed by the opening 33 at the bottom portion of the second accommodating part 32 and the flow channel forming part 35.

As shown in Fig. 4, a gap (a clearance) D corresponding to the flow channel for the second reagent 46 is formed between a back surface 36 of the flow channel forming part 35 of the multifunctional member 30 and the carrier 2 of the assay strip 1. The gap D is, for example, in the range of 0.01 mm to 1 mm. The second reagent 46 flows out from the opening 33 at the bottom portion of the second accommodating part 32 toward the carrier 2, and the second reagent 46 that has flowed out flows through the flow channel formed by the gap D and reaches at least above the assay region L1. The second reagent 46 that has reached the assay region L1 infiltrates the assay region L1 from the flow channel.

An absorption pad 6, which will be described later, is disposed at an end part on the downstream side of the assay strip 1. In the case member 20, a support part 22 that supports an end part of the assay strip 1 including the absorption pad 6 is formed at a position facing the absorption pad 6. The second accommodating part 32 of the multifunctional member 30 is disposed above the absorption pad 6. The support part 22 also supports the multifunctional member 30 via the absorption pad 6. In addition, in the case member 20, a support part 21 that supports a center part of the assay strip 1 is formed.

The assay strip 1 comprises the carrier 2, a liquid feeding pad 4, and the absorption pad 6. Then, the carrier 2 is fixedly supported on a back pressure-sensitive adhesive sheet 7.

The carrier 2 is a porous insoluble carrier for developing a sample, and comprises the assay region L1, the control region L2, and the color development region L3. In addition, the carrier 2 comprises a label holding pad 3. The label holding pad 3 is configured with a spotting region on which the sample is spotted. The color development region L3 is disposed on the downstream side of the assay region L1 in a case where the direction toward the assay region L1 with respect to the spotting region is set as the downstream side of the carrier 2. In the present example, the assay region L1, the control region L2, and the color development region L3 are line-like regions extending in a direction perpendicular to the development direction of the sample in the carrier 2.

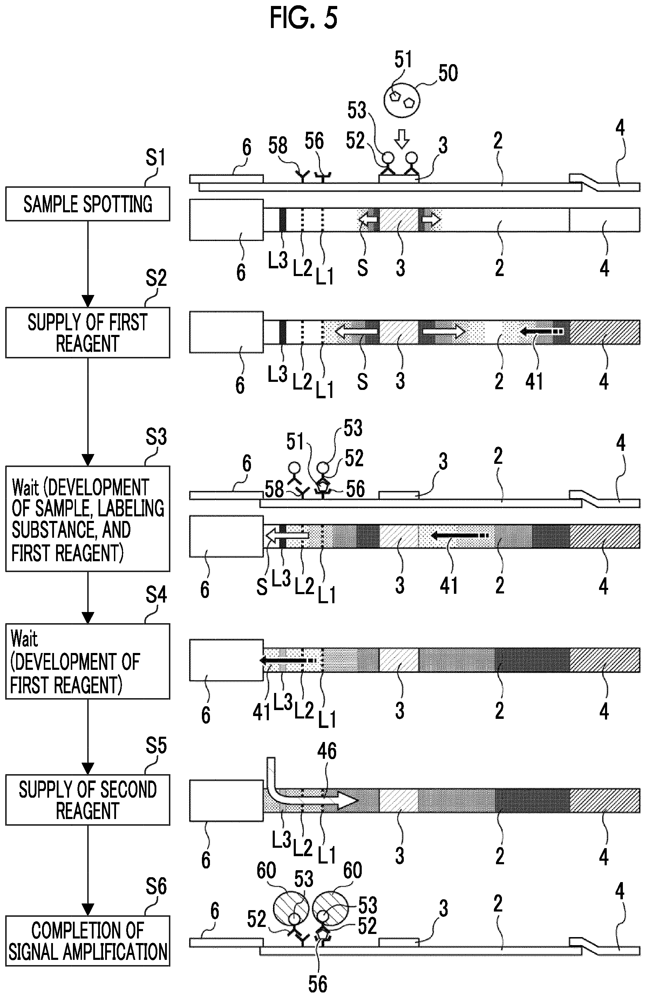

It shows a state in which the assay region L1, the control region L2, and the color development region L3 are expressed as lines, but these are not always expressed. Details will be described later, but before developing the sample 50 (see Fig. 5), the first reagent 41 (see Fig. 4), and the second reagent 46 (see Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. Accordingly, the assay region L1 enters visible. Since the color development of the assay region L1 is amplified by silver amplification, which will be described later, the assay region L1 develops a black color.

The control region L2 is also expressed as a line by increasing the color optical density in a case where the sample 50 is developed. Accordingly, the control region L2 enters visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

As shown in Fig. 5, a labeling substance 53 is fixed to the label holding pad 3. The labeling substance 53 is modified with a first binding substance 52 that specifically binds to a test substance 51 contained in the sample 50. The label holding pad 3 is fixed on the carrier 2 at a position facing the dropping port 16 of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that is used in an immune agglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, and the gold colloid is most preferable among them.

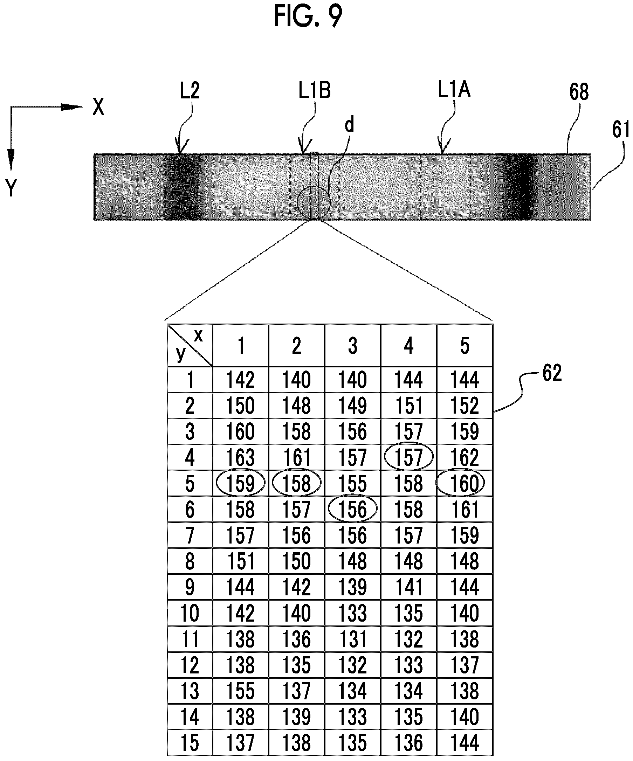

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bound to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be equal to or more than a preset reference. The assay region L1 is a region for confirming presence or absence of the test substance 51 by a labeling signal from the labeling substance 53 captured via the test substance 51. The test substance 51 detected by one cartridge 100 is not limited to one kind, and a plurality of assay regions L1 may be provided to simultaneously detect a plurality of different test substances 51. In a case where, for example, a first assay region L1A and a second assay region L1B are provided as the plurality of assay regions L1 (see Fig. 9), the first assay region L1A comprises a second binding substance 56 that specifically binds to the first test substance 51, and the second assay region L1B includes a second binding substance that specifically binds a second test substance different from the first test substance 51.

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be equal to or more than a preset reference. The control region L2 is a region for confirming the completion of the development of the sample 50 by the labeling signal from the labeling substance 53 captured via the first binding substance 52. Therefore, the control region L2 may be referred to as a confirmation region.

The first binding substance 52 that modifies the labeling substance 53 and specifically binds to the test substance 51 is a substance that specifically binds to the test substance 51, for example, in a case where the test substance 51 is an antigen, an antibody against the antigen, in a case where the test substance 51 is an antibody, an antigen against the antibody, in a case where the test substance 51 is a protein, a low-molecular-weight compound, or the like, an aptamer against the protein, the low-molecular-weight compound, or the like.

The second binding substance 56 that is fixed in the assay region L1 and specifically binds to the test substance 51 is a substance that specifically binds to the test substance 51, for example, in the case where the test substance 51 is an antigen, an antibody against the antigen, in the case where the test substance 51 is an antibody, an antigen against the antibody, in the case where the test substance 51 is a protein, a low-molecular-weight compound, or the like, an aptamer against the protein, the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, or the like.

For example, in a case where the test substance 51 is an influenza A virus or a biomarker thereof, an anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, 038-06925) is used as the first reagent 41, an aspect in which the color development region L3 is configured with a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

The liquid feeding pad 4 is disposed in contact with one end of the carrier 2 and the first reagent 41 is fed to the carrier 2 from the upstream side of the spotting region (configured with the label holding pad 3). In the liquid feeding pad 4, in a case where the first pressed part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary action.

The absorption pad 6 is disposed in contact with the other end of the carrier 2 and absorbs the sample 50, the first reagent 41, and the second reagent 46, which are developed on the carrier 2. The absorption pad 6 is also formed of a porous material.

In the present embodiment, the first reagent 41 and the second reagent 46 are amplification agents that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplification agents used for silver amplification, and the reaction between the first reagent 41 and the second reagent 46 using the labeling substance 53 as a catalyst is an amplification reaction. By the amplification reaction, silver particles 60 (see Fig. 5) having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, the silver particles 60 (see Fig. 5) are generated, and the generated silver particles 60 deposits on the labeling substance 53 using the labeling substance 53 as a nucleus. By depositing the silver particles on the labeling substance 53, the silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

### (First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. When an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of Fe²⁺ is preferably used.

A developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A are also can be used.

As the reducing agent, an ascorbic acid reducing agent is also preferable. The useful ascorbic acid reducing agent includes ascorbic acid, an analog thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D, L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second Reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic Method>

An immunochromatographic method will be described with reference to Fig. 5. Here, a case where the sample 50 includes the test substance 51, that is, on the premise that the sample 50 is positive will be described.

First, the sample 50 is spotted on the label holding pad 3 which is the spotting region (Step S1). The test substance 51 in the sample 50, which is spotted on the label holding pad 3, specifically binds to the first binding substance 52 that modifies the labeling substance 53 contained in the label holding pad 3. The sample 50 is developed toward the downstream side from the label holding pad 3 in the carrier 2 by the capillary action in the carrier 2. A part of the sample 50 is also developed toward the upstream side. The arrow S indicates a state in which the sample 50 is developed.

Next, the first reagent 41 is supplied (Step S2). The first reagent 41 is supplied from the liquid feeding pad 4. The first reagent 41 is supplied to the carrier 2 via the liquid feeding pad 4 and is developed toward the downstream side.

After that, the process waits until the first reagent 41 is developed toward the downstream side (Step S3 and Step S4). "Wait" shown in Fig. 5 means an action of waiting. The first reagent 41 is gradually developed toward the downstream side, and the sample 50 to be developed from the label holding pad 3 and the labeling substance 53 modified with the first binding substance 52 are developed toward the downstream side to be pushed by the first reagent 41 (Step S3).

The test substance 51 in the sample 50 that has been developed toward the downstream side and has reached the assay region L1 is captured by the second binding substance 56 of the assay region L1. That is, the labeling substance 53 is captured in the assay region L1 via the test substance 51 and the first binding substance 52. On the other hand, the labeling substance 53 that is not bound to the test substance 51 passes through the assay region L1 without being captured, and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed toward the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

The assay apparatus 110, as an example, the cartridge 100 is loaded in a state in which the spotting of the sample on the carrier 2 and the supply of the first reagent 41 and the second reagent 46 to the carrier 2 is started. That is, in the present example, the user performs pressing operation on the first pressed part 11 and the second pressed part 12 before loading the cartridge 100 into the assay apparatus 110. The assay apparatus 110 performs a determination of whether the sample is positive or negative based on an assay region image of the assay region L1 of the loaded cartridge 100, and presents a determination result.

As shown in Fig. 6, the assay apparatus 110 comprises an imaging unit 114, an illumination unit 115, a processor 120, and a memory 121 therein.

The imaging unit 114 captures an observation region including at least the assay region L1 and the control region L2, and outputs an observation image including the observation region to the processor 120. It is more desirable that the observation region includes the color development region L3.

The imaging unit 114 is an image sensor such as a complementary metal oxide semiconductor (CMOS) image sensor and a charge coupled device (CCD) image sensor. The imaging unit 114 is disposed, for example, at a position facing the observation window 18.

The illumination unit 115 is an example of a light source that illuminates the observation region. The illumination unit 115 is disposed on both sides with the imaging unit 114 interposed therebetween, and illuminates the observation region at a time of capturing. The illumination unit 115 has, for example, a semiconductor light source such as a light emitting diode as a light emitting element that emits illumination light.

The processor 120 integrally controls each part of the assay apparatus 110. An example of the processor 120 is a central processing unit (CPU) that performs various types of control by executing a program. The CPU functions as a control unit by executing the program. The memory 121 is an example of a memory connected to or built in the CPU as the processor 120. For example, a control program is stored in the memory 121. The processor 120 is realized by the CPU executing the control program.

The processor 120 performs the main determination, which is the determination of whether the sample is positive or negative, based on the captured image acquired via the imaging unit 114, that is, based on the assay region image of the assay region.

The main determination is performed by determining presence or absence of a change in the color development state of the assay region L1 based on the captured image. The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), or an aspect in which the density of the color changes (that is, a change in density). In the present example, since a line is expressed in the assay region L1 by the labeling substance 53 being captured in the assay region L1, or the silver amplification after capturing the labeling substance 53, presence or absence of the expression of the line in the assay region L1 is determined as presence or absence of the change in the color development state. A case where the line is expressed in the assay region L1 means that the sample 50 is positive, and a case where the line is not expressed means that the sample 50 is negative. The determination of the presence or the absence of the change in the color development state of the assay region L1 corresponds to the main determination of whether the sample 50 is positive or negative. A specific step of the main determination will be described later.

In a case where the processor 120 determines that the line is expressed in the assay region L1, that is, in a case where the processor 120 determines that the sample 50 is positive, the processor 120 displays an assay result "positive" on the monitor 119. In addition, in a case where the processor 120 determines that there is no change in the color development state in the assay region L1, that is, in a case where the sample 50 is determined as negative, the processor 120 displays an assay result "negative" on the monitor 119.

In addition to the control program, the memory 121 stores setting information that is preset in order for the processor 120 to perform various types of control. The setting information stores information that is necessary when the processor 120 determines the change in the color development state. An example of the setting information includes various threshold values for determining whether the sample is positive or negative and an exploration area for specifying the assay region L1 from the reference position.

Details of the main determination, which is the determination of whether the sample is positive or negative, in the assay apparatus 110 will be described.

In the assay apparatus 110, first, the processor 120 turns on the illumination unit 115 to illuminate the observation region exposed from the observation window 18, and causes the imaging unit 114 to perform capturing of the observation region in this state. Moreover, the processor 120 acquires the captured image from the imaging unit 114, and determines whether or not the line is expressed in the assay region L1 as the change in the color development state of the assay region L1 from the acquired captured image. As described above, in a case where the assay region L1 is expressed as a line, it means that the sample 50 is positive, and in a case where the line is not expressed, it means that the sample 50 is negative. Therefore, the processor 120 performs a determination of whether the sample 50 is positive or negative in accordance with the presence or the absence of the expression of the assay region L1.

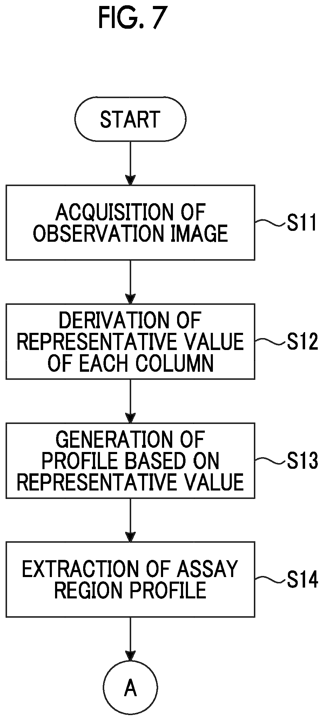

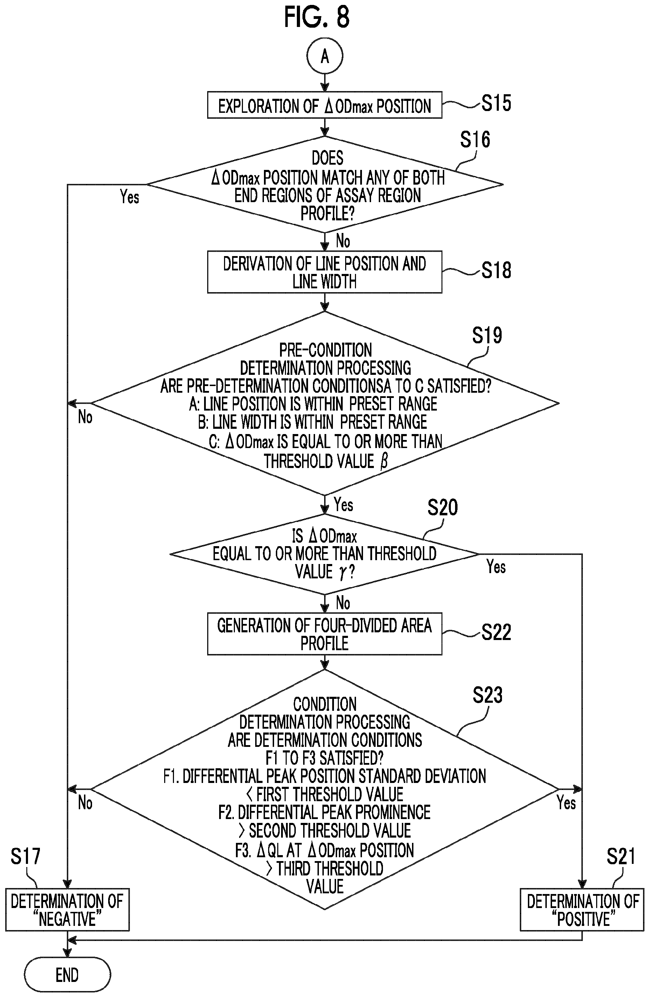

Figs. 7 and 8 show a processing procedure of the main determination. Fig. 9 is an explanatory diagram of a method of deriving a representative value in the processing procedure.

First, the processor 120 acquires an observation image 61 (see Fig. 9) by cutting out an observation region 68 (see Fig. 9) from the captured image output by the imaging unit 114 (Step S11).

The observation image 61 shown in Fig. 9 is shown as an actual image, not a schematic diagram. In the cartridge 100 of the embodiment described with reference to Figs. 1 to 6, the observation region includes the assay region L1, the control region L2, and the color development region L3, whereas the observation region 68 shown in the observation image 61 includes two first assay region L1A and second assay region L1B and the control region L2. That is, the observation image 61 shown in Fig. 9 is an image obtained by capturing an observation region of a cartridge having a configuration different from that of the cartridge 100 of the embodiment. The above-described cartridge 100 is a cartridge for assaying presence or absence of one type of test substance, but the cartridge in which the observation image 61 shown in Fig. 9 is captured is a cartridge for assaying presence or absence of two types of test substances. The technology of the present disclosure is not limited to a type of the cartridge.

In the cartridge for assaying the presence or the absence of the two types of test substances, the two types of test substances are, for example, influenza A type and influenza B type. In Fig. 9, the first assay region L1A is an assay region including a capture antibody for influenza A type, and the second assay region L1B is an assay region including a capture antibody for influenza B type. The width of the first assay region L1A, the second assay region L1B, and the control region L2 in the X direction is, for example, 1 mm, and the three regions of the first assay region L1A, the second assay region L1B, and the control region L2 are provided, for example, at 3 mm interval. Hereafter, in a case where it is necessary to distinguish a plurality of assay regions L1, a subdivided reference numeral of A or B is added to a reference numeral of the assay region L1, and in a case where it is not necessary to distinguish the plurality of assay regions L1, the reference numerals are simply referred to as assay regions L1.

The observation image 61 is an image in which a plurality of pixels are two-dimensionally arranged in a matrix shape. In a case where the length direction of the carrier 2 is defined as an X direction and the length direction of the line of the assay region L1, which intersects the X direction, is defined as a Y direction, a row direction indicates the X direction and a column direction indicates the Y direction. Fig. 9 shows, as an example, a pixel sequence 62 of 15th row and 5th column in a vicinity of a center of the second assay region L1B, which is a partial region of the observation image 61. The processor 120 converts the signal value for each pixel of the captured image output by the imaging unit 114 into a digital pixel value of 0 to 255. In Fig. 9, a pixel value is shown in each pixel in the shown pixel sequence 62. The pixel value of the observation image 61 corresponds to, for example, a brightness in accordance with an amount of received light incident on each pixel. Therefore, in the observation image 61, the pixel value is larger as the brightness is higher, and the pixel value is smaller as the brightness is lower. In a case where such pixels of the observation image 61 are represented by a level of an optical density (hereinafter, simply referred to as a density), the density is higher as the pixel value is smaller, and the density is lower as the pixel value is larger. Here, the pixel value and the density value can be converted by a preset conversion expression.

Next, the processor 120 derives a representative value of each column of the acquired observation image 61 (Step S12).

In the observation image 61 of the example shown in Fig. 9, a lower region of the second assay region L1B includes a portion (hereinafter, referred to as a high-density portion d) having a high density, such as a portion d surrounded by a circle. In a case where the sample 50 is positive for influenza B type, an average color optical density increases in substantially the entire second assay region L1B. Therefore, the same line as that in the control region L2 of the observation image 61 is expressed. On the other hand, it is presumed that the high-density portion d partially generated in the second assay region L1B is a portion in which the color optical density is increased due to stain or shadow such as non-specific adsorption of the labeling substance. Such a high-density portion d is an unnecessary stain that induces an erroneous determination in the main determination. In some cases, the high-density portion d shows a density that is abnormally higher than the color optical density in a case where the sample 50 is positive. Such high-density portion d has a small pixel value, and for example, in the pixel sequence 62 shown in Fig. 9 as an example, the high-density portion d shows a pixel value of approximately 140 or less. On the other hand, another portion of the second assay region L1B shows a pixel value of approximately more than 150.

The processor 120 sorts the pixels included in each column in order of the pixel values and derives, for example, a third highest pixel value as a representative value. In a case of the pixel sequence 62 shown in Fig. 9, the pixel value of the pixel surrounded by the circle is the third highest pixel value in each column.

In this way, by setting a pixel having a relatively high pixel value, that is, a pixel having a low density as the representative value in each column, an influence due to stain, shadow, or the like in the column direction is eliminated. As the representative value, here, the third highest pixel value in each column is extracted. However, a method of determining the representative value is not limited to this method. For example, residual pixels may be acquired by excluding pixels having a relatively small pixel value, that is, high-density pixels having a relatively high density in each column, and an average value, a center value, or the like of the residual pixels may be used as the representative value. Specifically, five pixels from a pixel having the highest density (minimum pixel value) to a fifth rank pixel in each column are selected as high-density pixels, the residual pixels are acquired except for the high-density pixels of the selected five pixels, and a similar step is performed. Here, the pixel having the relatively high density means that the density is high as compared with the density of the residual pixels. As described above, there is a correlation between the density value of the pixel and the pixel value, and the higher the density value is, the lower the pixel value is. Therefore, the pixel having the relatively high density corresponds to a pixel having a relatively small pixel value in terms of a pixel value.

In addition, in a case where it is not necessary to consider the influence due to stain, shadow, or the like, or in a case where the influence is not considered, an average value, a center value, or the like of all the pixels in each column may be derived as the representative value.

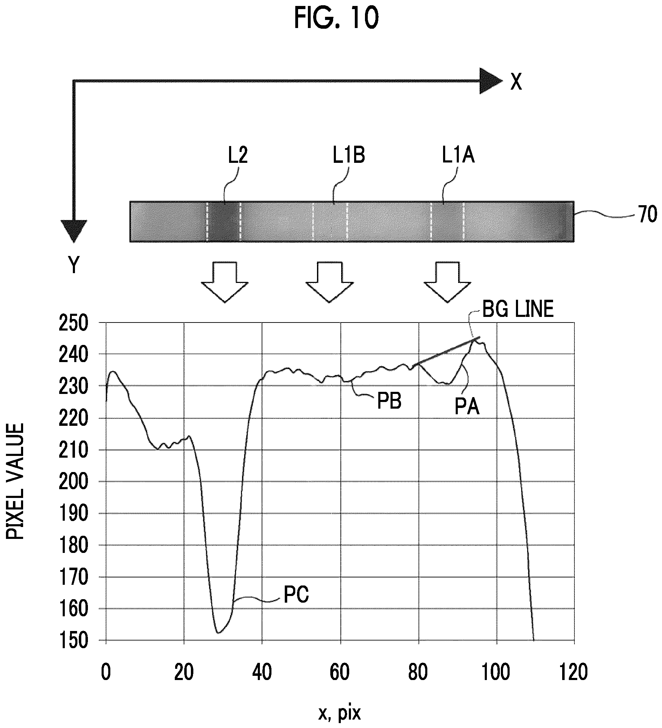

Next, the processor 120 generates an observation region profile of the pixel value in the row direction (that is, the X direction) by using the derived representative value (Step S13). Fig. 10 shows an example of an observation region profile generated from an observation image 70. In the observation region profile using the representative value of each column, a vertical axis is the pixel value, and a horizontal axis is a pixel position in the X direction. Since the observation image 70 is an image acquired for a sample different from that of the previously shown observation image 61, the observation image 70 is an image having a different gradation from a gradation of the observation image 61.

In the observation image 70 shown in Fig. 10, the control region L2 is clearly expressed in a line shape. That is, the line is expressed in the control region L2. In addition, the first assay region L1A is also expressed in a line shape, while the first assay region L1A is thinner than the control region L2. That is, a thin line is expressed in the first assay region L1A. On the other hand, the line is not expressed in the second assay region L1B. In the observation region profile of Fig. 10, each of regions indicated by PA, PB, and PC corresponds to the first assay region L1A, the second assay region L1B, and the control region L2, respectively. That is, in the observation region profile, PA refers to an assay region profile corresponding to the first assay region L1A, PB refers to an assay region profile corresponding to the second assay region L1B, and PC refers to a control region profile corresponding to the control region L2.

The processor 120 extracts the assay region profile from the observation region profile generated by using the representative value (Step S14). Specifically, the processor 120 extracts the assay region profile PA corresponding to the first assay region L1A and the assay region profile PB corresponding to the second assay region L1B from the observation region profile of Fig. 10.

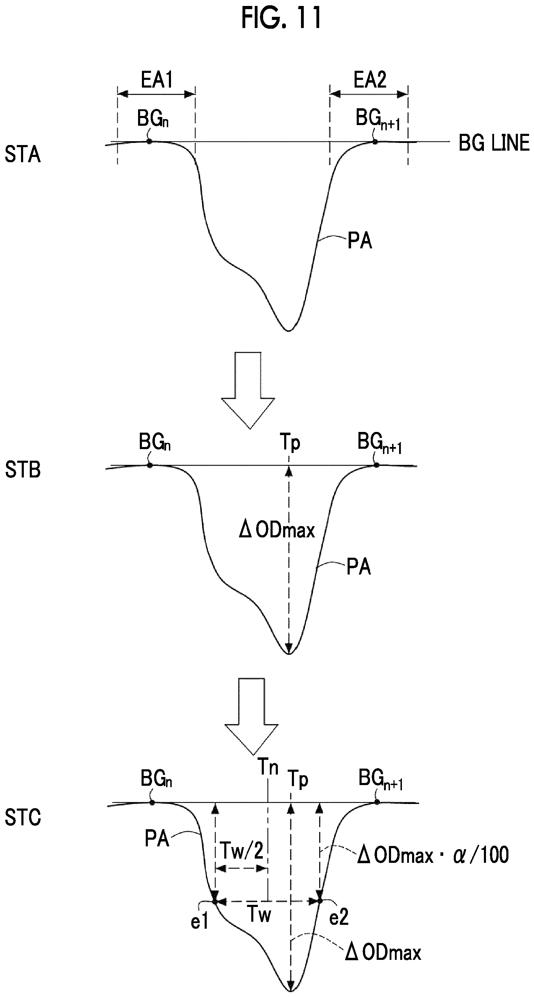

A specific method of Step S14 of extracting the assay region profile from the observation region profile generated based on the representative value will be described. Fig. 11 schematically shows a range including the assay region profile PA corresponding to the first assay region L1A in the observation region profile generated based on the representative value. Here, a procedure of determining whether the sample is positive or negative based on the presence or the absence of the expression of the line in the first assay region L1A will be described, and the same processing is executed for the second assay region L1B. Fig. 11 shows a step of extracting an assay region profile, exploring for a position showing a maximum density difference, and defining a line position and a line width of the line expressed in the first assay region L1A of the observation image 70.

First, in each exploration area set from a certain reference position in the observation region profile generated based on the representative value, points BGn and BGn + 1, which are both ends of the first assay region L1A for drawing a background line (hereinafter referred to as a BG line) are explored (see Step STA in Fig. 11). A certain reference is a preset position such as a known position or a previously detected position, for example, one end of the observation image 70 or one end of the control region L2. The positions of both ends of the first assay region L1A in the X direction from the reference position are known. The vicinities of the positions of both ends of the known first assay region L1A are defined as the exploration areas EA1 and EA2, and points having density values (pixel values) of preset order in the respective exploration areas EA1 and EA2 are denoted as BGn and BGn + 1, respectively. The preset order is appropriately set and is, in the exploration areas EA1 and EA2, to have the third highest density value (third lowest pixel value), to have the fifth highest density value (fifth lowest pixel value), or the like. The regions up to BGn and BGn + 1 thus determined are the assay region profile PA. In Fig. 11, the assay region profile PA is schematically displayed and the BG line is substantially horizontal, but as shown in Fig. 10, the actual BG line is not necessarily a horizontal line.

After the assay region profile PA is extracted, the processor 120 explores a maximum position (hereinafter, a ΔODmax position Tp) that is a row direction position Tp of a maximum density difference ΔODmax in which a density difference ΔOD between a profile density and the BG density in the assay region profile PA is maximum (Step S15). As shown in Step STB of Fig. 11, the assay region profile PA is a profile in a range of BGn to BGn + 1 searched in Step STA of Fig. 11. The maximum density difference ΔODmax means the maximum density of the assay region profile PA.

Next, the processor 120 determines whether or not the ΔODmax position Tp matches any of both end regions of the assay region profile PA (Step S16). Here, both ends of the assay region profile PA refer to BGn and BGn + 1, and both end regions refer to a range of BGn to BGn + 1, and a range of the exploration area EA1 of BGn and the exploration area EA2 of BGn + 1.

In a case where the position Tp of ΔODmax is located at both end regions of the assay region profile PA, it means that the assay region profile PA does not indicate a line expressed in the assay region L1 in a case where the sample is positive (hereinafter, a positive line). Therefore, in a case where the ΔODmax position Tp is located in any of both end regions of the assay region profile PA (Step S16; Yes), the processor 120 determines that the sample 50 is "negative" (Step S17), and ends the main determination.

On the other hand, in a case where the ΔODmax position Tp is not located in any of both end regions of the assay region profile PA (Step S16; No), the processor 120 derives the line position and the line width in the assay region profile PA (Step S18). At this point in time, it is not determined whether or not a positive line is expressed in the assay region L1, but the line position and the line width are defined on the assumption that a line is generated. The line position corresponds to the center position in the row direction of the assay region, and the line width corresponds to the width of the assay region.

As shown in Step STC of Fig. 11, the processor 120 explores the profile left and right from the ΔODmax position Tp, and derives a distance between two points e1 and e2 that are α% of ΔODmax as a line width Tw, and a central position of the line width Tw as a line position Tn in the first assay region L1A. α% is, for example, 50%, but can be appropriately determined to be 40%, 60%, or the like.

Next, the processor 120 executes pre-condition determination processing for determining whether or not the following pre-determination conditions A to C are satisfied (Step S19).
A: The line position Tn is within a preset range.
B: The line width Tw is within a preset range.
C: The maximum density difference ΔODmax is equal to or more than a preset threshold value β.

The preset range of the line position Tn of the condition A is, for example, a range in which the assay region L1 in the row direction defined from the above-described reference position can exist, and specifically, in a range of 10 mm to 11 mm from the reference position, or the like.

The preset range of the line width Tw of the condition B is a range of the designed assay region width (line width) Xw ± Δw, for example, in a case where the design value of the line width is 1 mm, a range of 0.8 mm to 1.2 mm, or the like.

The threshold value β of the condition C (corresponding to a fourth threshold value in the claims) is a value preset as a value that can be regarded as being certainly negative in a case where the threshold value β is less than β. As an index for detecting the change in the luminescent state, the value is appropriately determined in accordance with the principle of luminescence, the sensitivity of the imaging element, or the like.

The processor 120 determines that the pre-determination conditions are satisfied in a case where all the conditions A to C are satisfied, and the pre-determination conditions are not satisfied in a case where any one of the condition A, the condition B, or the condition C is not satisfied. In a case where it is determined that the pre-determination condition is not satisfied (Step S19: No), the processor 120 determines that the sample 50 is "negative" (Step S17), and ends the main determination.

On the other hand, in a case where it is determined that the pre-determination condition is satisfied (Step S19: Yes), the processor 120 determines whether or not ΔODmax is equal to or more than a threshold value γ (Step S20). Here, the threshold value γ (corresponding to a fifth threshold value in the claims) is set to a value that is larger than the threshold value β and can be regarded as a value at which ΔODmax is clearly expressed as a line. Therefore, in a case where it is determined that ΔODmax is equal to or more than the threshold value γ (Step S20: Yes), the processor 120 determines that the sample 50 is "positive" (Step S21), and ends the main determination. On the other hand, in a case where ΔODmax is less than the threshold value γ (Step S20: No), the processor 120 generates a four-divided area profile for the observation image (Step S22).

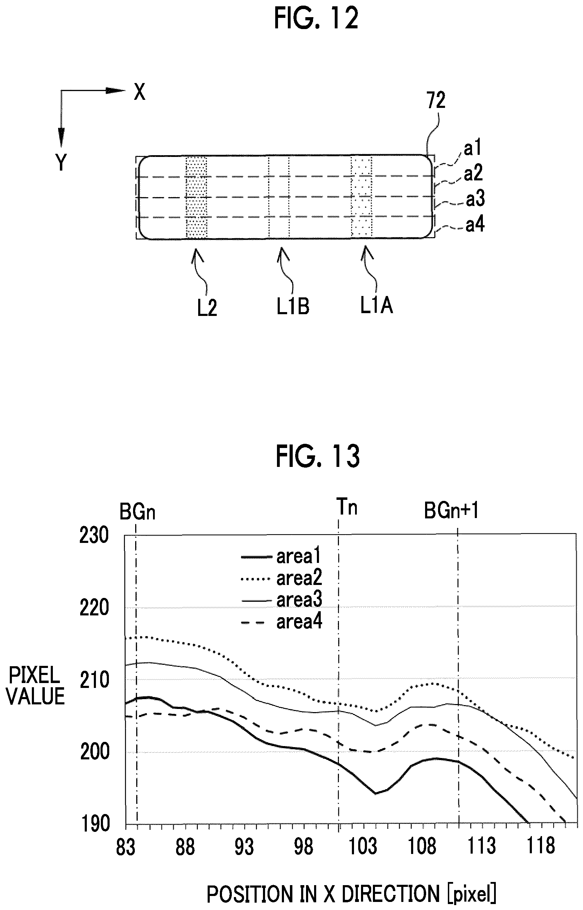

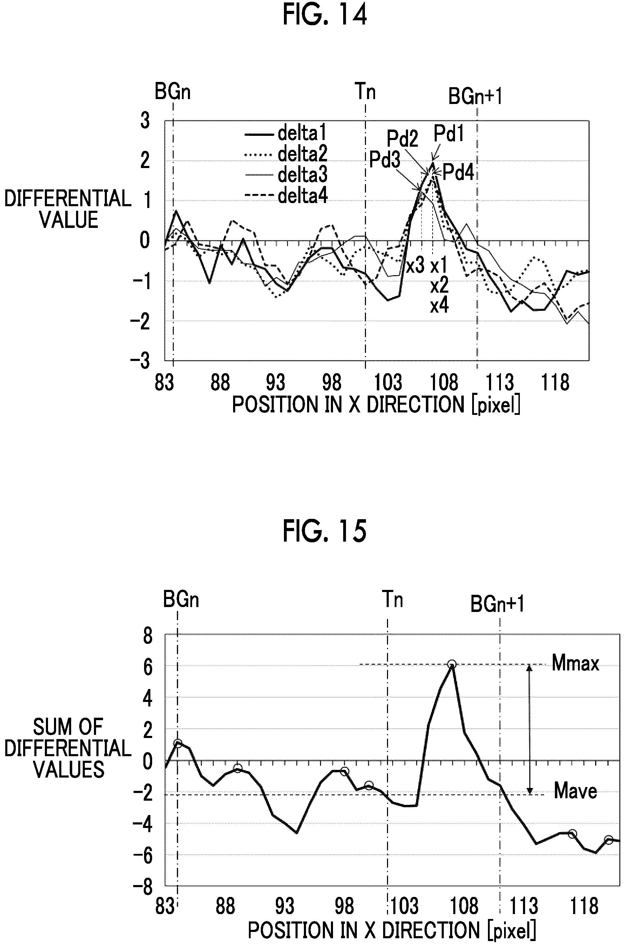

Here, the generation of the four-divided area profile will be described. Fig. 12 shows a schematic observation image 72. As shown in Fig. 12, the processor 120 divides the observation image 72 into four areas a1 to a4 extending in the row direction. Then, the processor 120 generates an area profile in the row direction for each of the areas a1 to a4. Specifically, an average value or a center value of each column in the areas a1 to a4, which is obtained by using at least a part of a plurality of pixels included in each of the columns is derived, and an area profile in the row direction for each of the areas a1 to a4 is generated by using the derived average value or the derived center value. Fig. 13 shows an example of area profiles area 1 to area 4 generated by dividing the assay region image into four parts. The area profiles area 1 to area 4 shown in Fig. 13 are partial regions of the observation image 72 shown in Fig. 12 and are profiles of regions including the first assay region L1A. In Fig. 13, the positions BGn, BGn + 1, and Tn in the row direction (the X direction) correspond to the positions of the same reference numerals shown in Fig. 11 (the same applies to Figs. 14 to 16). At least a part of a plurality of pixels included in each of the columns may be used for deriving the average value or the center value of each column in each of the areas a1 to a4, but half or more of the plurality of pixels included in each of the columns is preferably used and all of them is more preferably used. In addition, in the present example, the observation image is divided into four areas a1 to a4, but the number of divisions may be equal to or more than 2 and is not limited to 4.

Next, the processor 120 executes the condition determination processing using the area profiles area 1 to area 4 derived for each of the areas a1 to a4 (Step S23). As the condition determination processing, the processor 120 executes at least one of the first condition determination processing, the second condition determination processing, or the third condition determination processing for determining whether or not the value derived based on the area profiles area 1 to area 4 satisfies the preset condition. Then, in a case where there is condition determination processing in which even one of the executed condition determination processing does not satisfy the condition (Step S23: No), the processor 120 determines that the sample 50 is "negative" (Step S17). On the other hand, in a case where all the conditions of the executed condition determination processing are satisfied, the processor 120 determines that the sample 50 is "positive" (Step S21). Here, as an example, a case where all of the first condition determination processing to third condition determination processing are executed will be described.

The first condition determination processing is condition determination processing for determining whether or not the first condition F1 is satisfied. In the first condition determination processing, first, the processor 120 generates differential area profiles delta 1 to delta 4 (see Fig. 14) obtained by differentiating the area profiles area 1 to area 4 (see Fig. 13) for each area. Next, as shown in Fig. 14, the processor 120 derives positions (hereinafter, referred to as differential peak positions) x1 to x4 in the row direction indicating highest maximal values Pd1 to Pd4 in each of the differential area profiles delta 1 to delta 4. Then, the processor 120 derives a standard deviation of each of the derived differential peak positions x1 to x4 (differential peak position standard deviation), and determines whether or not to satisfy the first condition F1 that the standard deviation is less than the preset first threshold value. The fact that the standard deviation of the differential peak positions of the plurality of areas is less than the first threshold value means that a rate of match the differential peak positions is high. In a case where the differential peak positions match in the plurality of areas, there is a high possibility that a line extending in the column direction is expressed in the assay region. On the contrary, in a case where the first condition F1 is not satisfied, it means that the line is not expressed. Therefore, in a case where the first condition F1 is not satisfied, the processor 120 determines that the sample 50 is "negative".

The second condition determination processing is condition determination processing for determining whether or not the second condition F2 is satisfied. In the second condition determination processing, the processor 120 generates the differential area profiles delta 1 to delta 4 (see Fig. 13) obtained by differentiating the area profiles area 1 to area 4 (see Fig. 14) for each area, adds the differential area profiles delta 1 to delta 4, and generates an addition differential area profile (see Fig. 15). Then, the processor 120 derives a difference between a highest maximal value in an added addition differential area profile and an average value of maximal values other than the highest maximal value, and determines whether or not a second condition F2 that the difference is larger than a preset second threshold value is satisfied. In Fig. 15, the maximal value of the addition differential area profile is marked with a circle (O). Among these maximal values, the highest maximal value Mmax appearing at the position 107 pixel in the X direction and the average value Mave of the other six maximal values are derived. The range for extracting the maximal value is the same range on the right and left sides of the position in the X direction from the previously obtained line position Tn. For example, the range is a range of 1.8 mm in total, 0.9 mm each at the right and left sides of Tn as a center. The difference between the highest maximal value Mmax and the average value Mave of other maximal values is a measure of the prominence of the highest maximal value, and is hereinafter referred to as a differential peak prominence. The fact that the differential peak prominence is larger than the second threshold value means that there is a high possibility that the line is expressed in the assay region. On the contrary, in a case where the second condition F2 is not satisfied, it means that the line is not expressed. Therefore, in a case where the second condition F2 is not satisfied, the processor 120 determines that the sample 50 is "negative".

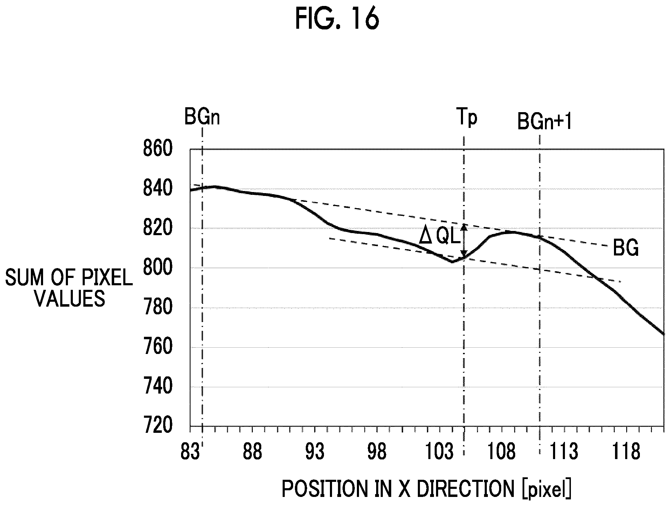

The third condition determination processing is condition determination processing for determining whether or not a third condition F3 is satisfied. In the third condition determination processing, the processor 120 adds the area profiles area 1 to area 4 (see Fig. 13) for each area to generate an addition area profile (see Fig. 16). Then, the processor 120 determines whether or not the third condition F3 is satisfied, in which a value at a preset position in the row direction in the addition area profile is larger than the third threshold value. In Fig. 16, BGn, BGn + 1, and Tp correspond to positions of the same reference numerals shown in Fig. 11. That is, BGn and BGn + 1 are both ends of the assay region profile PA previously explored in the profile using the representative value, and Tp is the ΔODmax position. In the addition area profile shown in Fig. 16, the processor 120 sets a straight line connecting a profile value (here, the sum of the pixel values) of the position BGn in the row direction (the X direction) and a profile value of the position BGn + 1 as the BG line. Then, the processor 120 derives the difference ΔQL between the profile value at the ΔODmax position Tp and the BG line as a value at a preset position in the row direction. The fact that the difference ΔQL is larger than the third threshold value means that a certain degree of density change has occurred in the ΔODmax position Tp obtained in the assay region profile PA generated using the representative value, and that there is a high possibility that the line is expressed. On the contrary, in a case where the third condition F3 is not satisfied, it means that the line is not expressed. Therefore, in a case where the third condition F3 is not satisfied, the processor 120 determines that the sample 50 is "negative".

As described above, in the condition determination processing using the area profiles area 1 to area 4, in a case where there is condition determination processing in which even one condition of the first condition determination processing to the third condition determination processing, which have been executed, is not satisfied (Step S23: No), the processor 120 determines that the sample 50 is "negative" (Step S17) and ends the main determination. On the other hand, in a case where all the conditions of the first condition determination processing to the third condition determination processing, which have been executed, are satisfied, the processor 120 determines that the sample 50 is "positive" (Step S21), and ends the main determination.

The processing procedure of the main determination in the assay apparatus 110 according to the present embodiment is as described above.

In the present embodiment, the processor 120 generates the observation region profile in the row direction by setting the pixel having the relatively high pixel value in each column of the observation image as the representative value. Moreover, the processor 120 derives the position of ΔODmax, the line position, and the line width to perform the pre-condition determination processing. By setting the pixel having the relatively high pixel value as the representative value, the influence can be eliminated, in which a pixel has a high density due to stain, shadow, or the like. Therefore, in the pre-condition determination processing, in a case where the pre-determination conditions A to C are satisfied, it is considered that the sample 50 is determined as positive and the main determination is ended. However, since the representative value is derived by using one specific pixel among the plurality of pixels configuring the column direction or only a part among the plurality of pixels, information (line continuity information) is lost, which is continuous in the column direction included in the line indicating an actual positivity. Therefore, the sample satisfying the pre-determination conditions A to C may include not only the positivity but also a false positivity.

In the present embodiment, the processor 120 divides the assay region image into a plurality of areas extending in the row direction, derives an average value or a center value of each column in the areas, and generates an area profile in the row direction for each area using the average value or the center value. The processor 120 executes condition determination processing using the area profile. As in the present embodiment, by executing the condition determination processing using the area profile, the main determination can be executed, which ensures the information about the line continuity, so that the determination result with the higher reliability can be obtained.

In the present embodiment, as the condition determination processing using the area profile, the processor 120 executes, using the differential area profile obtained by differentiating the area profile for each area, the first condition determination processing of determining whether or not the first condition F1 that the standard deviation of the differential peak positions of the plurality of areas is less than the first threshold value is satisfied. The fact that the differential peak positions substantially match in each area means that line continuity is present in the column direction. By determining presence or absence of such line continuity, a sample that does not have line continuity can be prevented from being erroneously determined as positive.

In the present embodiment, as the condition determination processing using the area profile, the processor 120 adds the differential area profiles for each area, and executes the second condition determination processing of determining whether or not the second condition F2 that a difference between a highest maximal value in an added addition differential area profile and an average value of maximal values other than the highest maximal value is larger than the second threshold value is satisfied. The fact that the differential peak prominence in the addition differential profile is larger than the second threshold value means that positions of the highest maximal values in a plurality of area profiles substantially match each other, which also means that the line continuity is also present in this case. Therefore, by determining the presence or the absence of the line continuity even in the second condition determination processing, the sample that does not have line continuity can be prevented from being erroneously determined as positive.

In the present embodiment, as the condition determination processing using the area profile, the processor 120 adds the area profiles for each area, and executes the third condition determination processing of determining whether or not the third condition F3 that the value at the preset position in the row direction in the addition area profile is larger than the third threshold value is satisfied. For example, the ΔODmax position in a representative value profile is set as the preset position in the row direction. In this case, in a case where the profile value in the addition area profile is larger than the third threshold value, the expression of the line is secured. Therefore, the sample that does not have line continuity can be prevented from being determined as positive by eliminating the sample that does not satisfy the third condition F3 in the third condition determination processing.

As described above, the processor 120 may be configured to execute a combination of only one or two of the first condition determination processing to the third condition determination processing. However, as in the present embodiment, in a case where the processor 120 executes all of the first condition determination processing, the second condition determination processing, and the third condition determination processing as the condition determination processing using the area profile, a result with the higher reliability can be obtained as compared with a case where only one or two processing are executed.

In the above-described embodiment, as a hardware structure of a processing unit that executes various pieces of processing by the processor 120, various processors shown below can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to execute specific processing.

One processing unit may be configured by one of the various processors or a combination of two or more processors of the same type or different types (for example, a combination of a plurality of FPGAs and/or a combination of a CPU and an FPGA). In addition, a plurality of processing units may be configured with one processor.

As an example in which a plurality of processing units are configured into a single processor, there is a form in which a single processor is configured by a combination of one or more CPUs and software, and this processor functions as a plurality of processing units. A second example of the configuration is an aspect in which a processor that implements the functions of the entire system including a plurality of processing units using one integrated circuit (IC) chip is used. A representative example of this aspect is a system on chip (SoC). Accordingly, various processing units are configured using one or more of the above-described various processors as a hardware structure.

Furthermore, specifically, an electric circuit (circuitry) obtained by combining circuit elements, such as semiconductor elements, can be used as the hardware structure of the various processors.

In the above-described embodiment, as the presence or the absence of a change in the color development state of the assay region L1 of the carrier 2 provided in the cartridge 100, the presence or the absence of the expression of the line by the gold colloid that is the label captured in the assay region L1 or by amplifying the captured gold colloid with silver ions is determined. The change in the color development state of the assay region L1 of the carrier 2 is not limited to the expression of a line by the gold colloid or by amplifying the gold colloid with silver ions. For example, using an enzyme label that generates chemiluminescence by contact with a luminescent reagent, the labeling may be the expression of a line by chemiluminescence generated by reacting the enzyme label captured in the assay region L1 with the luminescent reagent. In addition, using a fluorescent label, the labeling may be the expression of a line by fluorescence generated by irradiation of the fluorescent label captured in the assay region L1 with the excitation light.

The disclosure of Japanese Patent Application No. 2022-046017 filed on March 22, 2022 is incorporated herein by reference in its entirety.

All cited documents, patent applications, and technical standards described in the specification are incorporated by reference in the specification to the same extent as in a case where each individual cited document, patent application, or technical standard is specifically and individually indicated to be incorporated by reference.

## Claims

1. An immunochromatographic assay apparatus comprising:
a loading part into which a cartridge is attachably and detachably loaded, the cartridge including a carrier having a spotting region where a sample is spotted and an assay region where a color development state changes depending on whether the sample is positive or negative;
an image sensor that captures an observation region including the assay region and outputs an observation image including the observation region; and
a processor that performs a main determination, which is a determination of whether the sample is positive or negative, based on an assay region image of the assay region captured by the image sensor,
wherein in a case where a development direction of the sample in the carrier is set as a row direction and a direction intersecting the row direction is set as a column direction, the assay region is a line-like region extending along the column direction,
the assay region image is an image in which a plurality of pixels are two-dimensionally arranged in a matrix shape,
the processor divides the assay region image into a plurality of areas extending in the row direction, derives an average value or a center value in each column in the areas using at least a part of the plurality of pixels included in each of the columns, and generates an area profile in the row direction for each of the areas using the derived average value or center value, and
executes, in the main determination, condition determination processing using the area profile generated for each of the areas.

2. The immunochromatographic assay apparatus according to claim 1,
wherein the processor executes, as the condition determination processing, at least one of first condition determination processing, second condition determination processing, or third condition determination processing for determining whether or not a value derived based on the area profile satisfies a preset condition, and determines that the sample is negative in a case where even one of the conditions is not satisfied in the executed condition determination processing, or determines that the sample is positive in a case where all the conditions are satisfied in the executed condition determination processing,
the first condition determination processing is condition determination processing, in which a differential area profile obtained by differentiating the area profile for each of the areas is used, a position in the row direction indicating a highest maximal value in the differential area profile is derived for each of the areas, a standard deviation at a plurality of derived positions in the row direction is derived, and whether or not a first condition that the standard deviation is less than a preset first threshold value is satisfied is determined,
the second condition determination processing is condition determination processing, in which the differential area profile for each of the areas is added, a difference between a highest maximal value in an added addition differential area profile and an average value of maximal values other than the highest maximal value is derived, and whether or not a second condition that the difference is larger than a preset second threshold value is satisfied is determined, and
the third condition determination processing is condition determination processing, in which the area profile for each of the areas is added, and whether or not a third condition that a value at a position in a row direction preset in an addition area profile is larger than a third threshold value is satisfied is determined.

3. The immunochromatographic assay apparatus according to claim 2
wherein the processor executes, as the condition determination processing, all of the first condition determination processing, the second condition determination processing, and the third condition determination processing.

4. The immunochromatographic assay apparatus according to any one of claims 1 to 3,
wherein the processor determines, in the main determination, a representative value of each of the columns of the observation image before the condition determination processing, generates an observation region profile in the row direction based on the representative value of each of the columns, and extracts an assay region profile corresponding to the assay region from the generated observation region profile,
determines that the sample is negative in a case where a maximum position, which is a position in the row direction indicating a maximum density in the assay region profile, is located in any of both end regions of the assay region profile,
derives a center position of the assay region in the row direction and a width of the assay region, which are determined based on the maximum density and the maximum position, in a case where the maximum position is not located in any of both the end regions,
executes pre-condition determination processing, in which whether or not a pre-determination condition that the maximum density is equal to or more than a preset fourth threshold value and each of the center position and the width is within a preset range is satisfied is determined, and
determines that the sample is negative in a case where the pre-determination condition is not satisfied, or executes the condition determination processing in a case where the pre-determination condition is satisfied.

5. The immunochromatographic assay apparatus according to claim 4,
wherein the processor determines, in the main determination, whether or not the maximum density is equal to or more than a preset fifth threshold value larger than the fourth threshold value before the condition determination processing is executed in a case where it is determined that the pre-determination condition is satisfied in the pre-condition determination processing, and determines that the sample is positive in a case where the maximum density is equal to or more than the fifth threshold value, or executes the condition determination processing in a case where the maximum density is less than the fifth threshold value.
